# EUROPEAN PATENT APPLICATION

(11) **EP 2 952 104 A1**
(43) Date of publication of application: **09.12.2015**
(21) Application number: 14171308.1
(22) Date of filing: 05.06.2014
(51) Int. Cl.: A23L 1/30, A61K 31/00

(54) **Composition and method for preventing damage of human cells by ionising radiation**

(71) Applicant: Böhm, Kirsten, 10117 Berlin (DE)
(72) Inventor: The designation of the inventor has not yet been filed
(74) Representative: Gulde & Partner

(57) **Abstract**

The present invention relates to a composition comprising a combination of one or more dietary carotenoids or their cis-trans isomers, preferably at least lycopene, with vitamins E and C, or with derivatives of vitamins E and C, for use in the prevention of damage of human cells, especially lymphoid cells, by ionising radiation. Particularly, the composition of the invention efficiently protects human cells against gamma radiation doses up to 5000 Gray (Gy). The composition of the invention is especially useful to be administered to patients undergoing radiation therapy in cancer treatment, to people living in the vicinity of a nuclear power station, after a nuclear based accident and/or improper use of nuclear material and to flight staff on aeroplanes and spacecraft.

## Description

The present invention relates to a composition comprising a combination of one or more dietary carotenoids or their cis-trans isomers with vitamins E and C, or with derivatives of vitamins E and C, for use in the prevention of damage of human cells, especially lymphoid cells, by ionising radiation, preferably gamma radiation. In one embodiment of the invention the composition comprises at least lycopene or mixtures of lycopene with one or more dietary carotenoids. In another embodiment of the invention the composition comprises at least lycopene or mixtures of lycopene with one or more dietary carotenoids other than β-carotene. Particularly, the composition of the invention efficiently protects human cells against radiation doses up to 5000 Gray (Gy). The composition of the invention is especially useful to be administered to patients undergoing radiation therapy in cancer treatment, to people living in the vicinity of a nuclear power station, after a nuclear based accident and/or improper use of nuclear material and to flight staff on aeroplanes and spacecraft.

Ionising radiation is composed of electromagnetic radiation and energetic sub-atomic particles e.g. ions or atoms. The threshold for electromagnetic radiation lies in the UV region of the electromagnetic spectrum. Gamma rays are one of the most energetic forms of electromagnetic radiation with the shortest wavelength and highest energy.

Carotenoids such as ß-carotene and lycopene are well known as antioxidants which protect against sunlight induced skin damage. They are efficient singlet oxygen quenchers, but their aggregation can markedly reduce the singlet oxygen quenching efficiency. Free radical interactions with carotenoids lead to at least three processes, electron and hydrogen atom transfer and adduct formation. The most studied is the electron transfer where the carotenoid loses an electron to become a radical cation. The reactivity/lifetime of such carotenoid radicals may lead to a switch from anti- to pro-oxidant behaviour (see F. Böhm et al., Mol. Nutr. Food Res. 2012, 56, 205-216). Vitamins E and C are also known as radical scavengers. For example, DE 202013005637 U1 describes a composition containing tocotrienols for treating erythemae caused by sun light, radioactivity or heat.

A combination of lycopene with vitamin E is described in WO 98/057622 A1 which inhibits the oxidation of LDL (low density lipoproteins) in serum. WO 03/068202 A1 discloses the use of lycopene in combination with vitamin E and/or C for prevention of angiogenesis-associated pathologies. To protect against UV radiation DE 4020874 A1 describes a soft drink which contains a carotenoid mixture, optionally together with vitamin C and/or vitamin E. WO 2001089542 A2 describes a method for improving cell protection against free radicals by administering a composition comprising ß-carotene, lycopene, vitamin E, vitamin C and an extract of Vitis vinifera.

It was the objective of the present invention to provide a composition for protection of human cells against ionising radiation, especially gamma radiation, which efficiently minimizes the damage caused by this type of radiation. It was a particular objective to provide a composition for protection of fast-growing cells, in particular lymphoid cells. An additional objective is the provision of a method for protecting humans against ionising radiation.

The inventors of the present invention have found that a combination of one or more dietary carotenoids, preferably at least the carotenoid lycopene, with vitamins E and C is protecting human cells against ionising radiation, especially gamma radiation, by about a factor of 4 to 5 compared to unprotected cells (see Table 1). A protection factor of 5 means that the damage caused by the radiation is reduced to 20 %. This preferably relates to radiation doses from 25 to 5000 Gy, especially from 500 to 5000 Gy. Preferably, the composition of the present invention comprises at least lycopene or mixtures of lycopene with one or more other dietary carotenoids, preferably not β-carotene. The composition of the present invention does also not contain a plant extract of Vitis vinifera.

Hence, the subject matter of the present invention is a composition comprising a combination of one or more dietary carotenoids or their cis-trans isomers, preferably at least lycopene, with vitamins E and C, or derivatives of vitamins E and C, as active ingredients for use in the prevention of damage of human cells by ionizing radiation. The composition of the present invention is intended for oral or parenteral administration, preferably for oral administration. The term vitamin E as used herein includes racemic vitamin E (D,L-α-tocopherol) or natural vitamin E, as well as derivatives thereof which have biological vitamin E activity, e.g. carboxylic acid esters, such as vitamin E acetate, propionate, butyrate or succinate. The term vitamin C as used herein includes derivatives thereof which have biological vitamin C activity, e.g. esters and salts, such as sodium ascorbate, sodium ascorbyl phosphate and ascorbyl palmitate.

The carotenoids which can be used according to the present invention comprise all dietary carotenoids, in the all trans or various cis isomeric forms. Mainly they comprise lycopene, astaxanthin, canthaxanthin, zeaxanthin, meso-zeaxanthin, cryptoxanthin, alpha-carotene, cis-beta carotenes, echinone, 7,7 dihydro beta carotene, lutein, beta-apo-carotenals. But also all other dietary carotenoids which appear in a normal diet can be used.

Preferably the radiation damage which is prevented by the composition of the present invention is cell membrane damage. The cells which are protected by the composition of the present invention are all fast-growing cells. In a preferred embodiment of the invention lymphoid cells are protected. But also skin cells, mucous cells, macula cells, bone marrow cells, blood cells, endothelial cells, gland cells, prostate cells, intestinal cells and supporting cells can advantageously be protected against ionising radiation by administering the composition of the invention.

According to the invention the lycopene used can be produced e.g. by extraction of oleoresin of tomatoes, by fermentation of algae or fungi or by synthetic preparation methods. In a specific aspect of the present invention tomato oleoresin which contains the lycopene can be included in the composition of the present invention to exhibit together with vitamins E and C the described effects. For this case the amount of tomato oleoresin has to be chosen such that the respective efficient amount of lycopene in the composition is ensured.

In a specific embodiment of the invention the composition which is intended for oral administration can be a pharmaceutical formulation or dietary composition which is preferably used as solid or liquid preparation. Special application or dosage forms are for instance capsules (e.g. hard or soft shell gelatine capsules), tablets, powders, pills, granules, sachets, oral suspensions, emulsions or solutions, syrups or elixirs.

For daily oral consumption by a human adult it is preferred to administer about 15 to 90 mg of lycopene, more preferred 30 to 90 mg. With regard to vitamins E and C it is preferred to administer about 100 to 3000 mg of each vitamin per day, more preferred 500 to 1000 mg. These amounts can be administered in one, two or more dosage units, wherein an application more than twice a day depends on the patient/consumer compliance.

According to a preferred embodiment of the present invention a dosage unit of said oral pharmaceutical formulation or dietary composition contains from about 15 to 45 mg of lycopene, related to the total weight of the composition, preferably about 15 to 30 mg of lycopene. In another preferred embodiment a dosage unit of said oral pharmaceutical formulation or dietary composition contains from about 100 to 1500 mg of vitamin E, related to the total weight of the composition, preferably about 100 to 1000 mg of vitamin E. In yet another preferred embodiment a dosage unit of said oral pharmaceutical formulation or dietary composition contains from about 100 to 1500 mg of vitamin C, related to the total weight of the composition, preferably about 100 to 1000 mg of vitamin C.

The composition of the invention can optionally include auxiliary ingredients which are from health aspects acceptable for oral or parenteral administration and well known in the art. Such conventional auxiliary ingredients can be organic and/or inorganic materials. Suitable ingredients include water, PBS, gelatine, gum Arabic, lactose, starch, magnesium stearate, talc and/or vegetable oils. Additionally, additives such as stabilizers, emulsifying agents, buffers, flavouring agents and preservatives may be added in accordance with accepted practices of pharmaceutical or dietary compounding.

Another object of the present invention is the use of a combination of one or more dietary carotenoids or their cis-trans isomers, preferably at least lycopene, with vitamins E and C, or with derivatives of vitamins E and C, as active ingredients for manufacturing a pharmaceutical or dietary composition for prevention of damage of human cells by ionising radiation which can be administered orally or parenterally, e. g. as injection or infusion solution. The preparation of such compositions comprising one or more carotenoids and vitamins E and C can be done with conventional methods by mixing the components and optionally adding the respective auxiliary ingredients as explained above to obtain a stable solid or liquid oral composition or by adding e. g. PBS and further common auxiliary ingredients to obtain an infusion or injection solution.

The present invention also concerns a method of preventing damage of human cells by ionising radiation which comprises administering to a subject in need of such prophylaxis an effective amount of a composition comprising a combination of one or more dietary carotenoids or their isomers, preferably at least lycopene or mixtures of lycopene with one or more dietary carotenoids, with vitamins E and C, or derivatives of vitamins E and C. In an embodiment of the invention the composition is administered before, during and/or immediately after exposure to ionising radiation, preferably before and/or during the exposure.

A major aspect of the present invention is the finding that cell damage by gamma radiation depends on the oxygen concentration in the respective tissue. The cell protection against damage is increased as the oxygen concentration in the cells is reduced. Accordingly, in addition to the administration of the combination of one or more dietary carotenoids or their cis-trans isomers, preferably at least lycopene or mixtures of lycopene with one or more dietary carotenoids, vitamin E and vitamin C the variation of the oxygen concentration in the exposed tissue can be a further measure to both increase or prevent cell damage by ionising radiation.

For example, in advance of a radio therapeutic treatment of a solid tumour or malignant disorders of the blood system such as e.g. lymphomas it may be especially useful to reduce the oxygen content of the exposed non-cancerous tissue and/or to increase the oxygen concentration in tumour tissue. For example, carbon dioxide can be used to vary the composition and effects of carotenoid/oxygen mixtures so as to have minimal effects on cancerous tissue but to protect the non-cancerous tissue.

The following examples are offered to illustrate the effects of the composition of the present invention and of the oxygen reduction in the respective tissues. The examples are not intended to be limiting in any respect.

### Example 1: Effects of a combination of lycopene, vitamin E and vitamin C on lymphoid cells at different gamma radiation doses

Human blood was taken from volunteers who had either supplemented their diet with a large dose of lycopene [90mg/day] and, in addition, vitamins C and E [3000mg each/day] or had relatively near zero intake of lycopene (i.e. avoiding all tomato-based foods) and vitamin C and E, both for 4 weeks.

Lymphoid cells were obtained from the blood via standard separation techniques. Samples with and without lycopene and vitamins E and C were exposed to gamma radiation from a ⁶⁰CO source (Foss Therapy Services Model 812) and irradiated with a range of doses (500-7500 Gy).

Cell membrane destruction leading to immediate cell death was shown by cell staining with eosin (2 µl 1% eosin to 50 µl cell suspension, 5 min). The results (s < 3.0) are based on the mean of at least 12 measurements and were corrected for the small percentage (< 2%) of stained cells due to the preparation technique. Significant differences with *P* < 0.01 or less (Mann-Whitney *U*-test) were observed between the percentage of stained cells with lycopene and vitamins E and C and without lycopene and vitamins E and C.

Table 1 compares the percentage of stained (dead) cells as a function of gamma radiation dose for both sets of cells, without and with the lycopene/vitamin E/vitamin C supplements. The Protection Factor (PF) is the ratio of the stained cell percentages.

**Table 1. Cell death as a function of radiation dose. Cell membrane destruction leading to immediate cell death was shown by cell staining with eosin.**

| γ-Radiation Dose /Gy | % stained cells with zero lycopene or vitamin C or E via supplementation | % stained cells with lycopene + vitamin C and E supplementation | Protection Factor (PF) |
|---|---|---|---|
| 500 | 7.10 | 1.39 | 5.11 |
| 1000 | 18.64 | 3.65 | 5.11 |
| 2000 | 43.81 | 8.93 | 4.91 |
| 5000 | 69.76 | 17.15 | 4.07 |
| 7500 | 86.23 | 67.33 | 1.28 |

### Example 2: Effect of oxygen concentration on the protection of human lymphoid cells at 2000 Gy gamma radiation dose

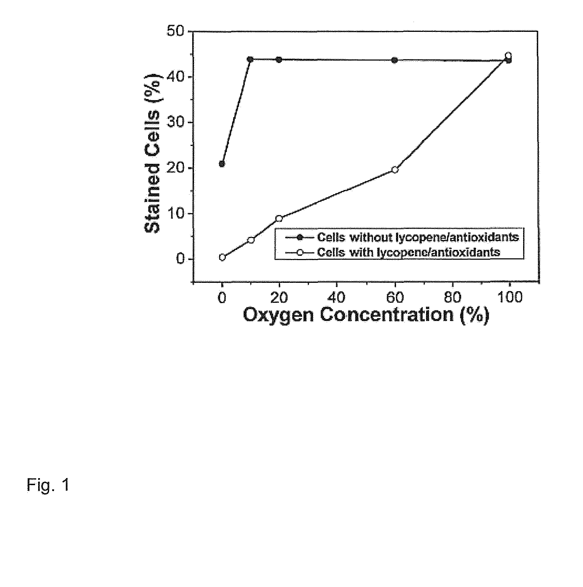

It has been shown that a reduction of the oxygen concentration increases the protection of the cells by the lycopene/ vitamin E / vitamin C combination. This important and unexpected finding is shown in Table 2 and Fig.1.

**Table 2**

| % Oxygen Concentration* | % stained cells with zero lycopene or vitamin C or E via supplementation | % stained cells - with lycopene + vitamin C and E supplementation | Protection Factor (PF) |
|---|---|---|---|
| 0 | 20.98 | 0.42 | 49.95 |
| 10 | 43.86 | 4.15 | 10.57 |
| 20 | 43.81 | 8.93 | 4.91 |
| 60 | 43.68 | 19.61 | 2.23 |
| 100 | 43.54 | 44.63 | 0.98 |

| | | | |
|---|---|---|---|
| *All percentages made to 100% with nitrogen | | | |

Cell death by 2000Gy γ-radiation as a function of oxygen concentration (using oxygen/nitrogen mixtures with all percentages made to 100% with nitrogen).
Cell membrane destruction, leading to immediate cell death, was shown by cell staining with eosin.

**Fig. 1** shows the plot of percentage of dead cells as a function of oxygen concentration at 2000 Gy gamma radiation.

## Claims

1. Composition comprising a combination of one or more dietary carotenoids or their cis-trans isomers with vitamins E and C, or with derivatives of vitamins E and C, as active ingredients for use in the prevention of damage of human cells by ionising radiation.

2. Composition according to claim 1 comprising at least lycopene or mixtures of lycopene with one or more dietary carotenoids.

3. Composition according to claim 1 or 2 comprising at least lycopene or mixtures of lycopene with one or more dietary carotenoids, other than β-carotene.

4. Composition according to any one of claims 1 to 3 comprising auxiliary ingredients which are acceptable for oral or parenteral administration.

5. Composition according to any one of claims 1 to 4, wherein the composition is an oral or parenteral pharmaceutical formulation or dietary composition.

6. Composition according to any one of claims 1 to 5, wherein the ionising radiation is gamma radiation.

7. Composition according to any one of claims 1 to 6, wherein the application forms of the composition are capsules, tablets, powders, pills or sachets, oral suspensions, oral emulsions or solutions, syrups or elixirs or injection or infusion solutions.

8. Composition according to any one of claims 1 to 7, wherein the human cells which are protected against damages by ionising radiation are lymphoid cells, skin cells, mucous cells, macula cells, bone marrow cells, blood cells, endothelial cells, gland cells, prostate cells, intestinal cells and supporting cells, preferably lymphoid cells.

9. Composition according to any one of claims 1 to 8, wherein the human cells are protected against damages caused by gamma radiation doses of up to 5000 Gy.

10. A method of preventing damage of human cells by ionising radiation which comprises administering to a subject in need of such prophylaxis an effective amount of a composition comprising a combination of one or more dietary carotenoids or their cis-trans isomers with vitamins E and C, or with derivatives of vitamins E and C, as active ingredients, the dietary carotenoids preferably comprising at least lycopene or mixtures of lycopene with one or more dietary carotenoids.

11. The method according to claim 10, wherein the composition is administered before, during and/or immediately after exposure to ionising radiation, preferably before and/or during the exposure.

12. The method according to claims 10 or 11, which comprises the variation of oxygen concentration in the tissue exposed to ionising radiation, preferably the increase of oxygen concentration in tumour tissue and/or decrease of oxygen content in the surrounding, non-cancerous, tissue before a radio therapeutic treatment.

13. Use of a combination of one or more dietary carotenoids or their cis-trans isomers with vitamins E and C, or with derivatives of vitamins E and C, as active ingredients for manufacturing a pharmaceutical or dietary composition for oral or parenteral administration preventing damage of human cells by ionising radiation, the dietary carotenoids preferably comprising at least lycopene or mixtures of lycopene with one or more dietary carotenoids.
